# EUROPEAN PATENT APPLICATION

(11) **EP 4 292 587 A1**
(43) Date of publication of application: **20.12.2023**
(21) Application number: 23179240.9
(22) Date of filing: 14.06.2023
(51) Int. Cl.: A61K 9/20, A61K 31/165

(54) **GUANFACINE HYDROCHLORIDE CONTAINING PHARMACEUTICAL PREPARATION**

(30) Priority: 15.06.2022 US 202263352282 P; 08.06.2023 JP 2023094806
(71) Applicant: Sawai Pharmaceutical Co., Ltd., Yodogawa-ku Osaka-shi Osaka 532-0003 (JP)
(72) Inventor: OKADA, Taro, Osaka, 5320003 (JP); KIMATA, Ryota, Osaka, 5320003 (JP); YOSHIHARA, Naoki, Osaka, 5320003 (JP)
(74) Representative: dompatent von Kreisler Selting Werner - Partnerschaft von Patent- und Rechtsanwälten mbB

(57) **Abstract**

An object of the present invention is to provide a guanfacine hydrochloride preparation in which an increase of related substances with time due to hydrolysis is suppressed. According to an embodiment of the present invention, a guanfacine hydrochloride preparation is provided containing one or more stability-improving additives selected from a group consisting of potassium dihydrogen phosphate, phosphoric acid, sodium dihydrogen phosphate anhydrous, carmellose, microcrystalline cellulose, carmellose calcium, low-substituted hydroxypropylcellulose, and hypromellose phthalate.

## Description

### TECHNICAL FIELD

An embodiment of the present invention relates to a guanfacine hydrochloride containing pharmaceutical preparation.

### BACKGROUND ART

A guanfacine hydrochloride containing pharmaceutical preparation (N-Amidino-2-(2,6-dichlorophenyl)acetamide monohydrochloride) is a selective α_{2A} adrenergic receptor agonist and has been used as a therapeutic for attention deficit hyperactivity disorder (AD/HD).

Guanfacine hydrochloride is a weakly basic drug with pH-dependent solubility and higher solubility at lower pH levels. pH-dependent dissolution is generally considered to be an undesired product-characteristic, and Patent Literature 1 discloses a pharmaceutical composition having minimized pH-dependency or pH-independent dissolution profiles.

In addition, Patent Literatures 2 and 3 disclose a guanfacine hydrochloride preparation effective in once-a-day administration that realizes an advantageous pharmacokinetic profile by blending poly (methacrylic acid, ethyl acrylate), which is a sustained-release polymer, at a specific ratio.

However, in Patent Literatures 1 to 3, the solubility of guanfacine hydrochloride or the pharmacokinetic profile of the guanfacine hydrochloride preparation are examined, and the stability of the guanfacine hydrochloride preparation is not examined. Furthermore, Patent Literatures 1 to 3 do not disclose that a hydrolysate of guanfacine hydrochloride increases with time, and no study has been made to suppress the increase of related substances of guanfacine hydrochloride.

### CITATION LIST

### PATENT LITERATURE

Patent Literature 1: Japanese Patent No. 4340840
Patent Literature 2: Japanese Patent No. 5726401
Patent Literature 3: Japanese laid-open patent publication No. 2009-502957

### SUMMARY OF INVENTION

### TECHNICAL PROBLEM

An object of an embodiment of the present invention is to provide a guanfacine hydrochloride preparation in which an increase of related substances with time due to hydrolysis is suppressed.

### SOLUTION TO PROBLEM

According to an embodiment of the present invention, there is provided a guanfacine hydrochloride preparation containing one or more stability-improving additives selected from a group consisting of potassium dihydrogen phosphate, phosphoric acid, sodium dihydrogen phosphate anhydrous, carmellose, microcrystalline cellulose, carmellose calcium, low-substituted hydroxypropylcellulose, and hypromellose phthalate.

0.5% by weight to 25% by weight of the stability-improving additive may be contained with respect to 100% by weight of the guanfacine hydrochloride preparation.

### ADVANTAGEOUS EFFECTS OF INVENTION

According to an embodiment of the present invention, there is provided a guanfacine hydrochloride preparation in which the increase of related substances with time due to hydrolysis is suppressed.

### DESCRIPTION OF EMBODIMENTS

Hereinafter, a guanfacine hydrochloride preparation according to the present invention will be described. In addition, the guanfacine hydrochloride preparation of the present invention is not to be construed as being limited to the description contents of the following embodiments and examples.

As a result of studies by the present inventors, it has been found that it is possible to suppress an increase of related substances of guanfacine hydrochloride by incorporating a specific stability-improving additive.

### [Guanfacine Hydrochloride Preparation]

The guanfacine hydrochloride preparation according to an embodiment of the present invention contains at least guanfacine hydrochloride and a stability-improving additive. In the present specification, the stability-improving additive is an additive capable of suppressing an increase of the related substances of guanfacine hydrochloride, and examples thereof include potassium dihydrogen phosphate, phosphoric acid, sodium dihydrogen phosphate anhydrous, carmellose, microcrystalline cellulose, carmellose calcium, low-substituted hydroxypropylcellulose, and hypromellose phthalate. These stability-improving additives may be contained in the guanfacine hydrochloride preparation alone or in a combination of two or more.

Carmellose is particularly preferred as a stability-improving additive. In addition, the guanfacine hydrochloride preparation according to an embodiment of the present invention contains one or more pharmaceutically acceptable additives in addition to the guanfacine hydrochloride and the stability-improving additive.

The guanfacine hydrochloride preparation according to an embodiment of the present invention contains 0.5% by weight to 25% by weight, preferably 0.9% by weight to 8% by weight of the stability-improving additive with respect to 100% by weight of the guanfacine hydrochloride preparation. The guanfacine hydrochloride preparation according to an embodiment of the present invention contains the above-described stability-improving additive within these ranges with respect to the guanfacine hydrochloride, thereby suppressing an increase of the related substances with time due to hydrolysis.

The guanfacine hydrochloride preparation according to an embodiment of the present invention can contain 1.14 mg, 2.28 mg, or 3.42 mg (1 mg, 2 mg, or 3 mg as guanfacine) of guanfacine hydrochloride per tablet. However, the content of guanfacine hydrochloride is not limited to these, and can be arbitrarily set within a range in which a therapeutic effect can be obtained.

Examples of one or more pharmaceutically acceptable additives contained in the guanfacine hydrochloride preparation according to an embodiment of the present invention can include but are not limited to excipients, disintegrants, sustained release agents, binders, lubricants, and colorants.

Examples of the excipient contained in the guanfacine hydrochloride preparation according to an embodiment of the present invention can include but are not limited to one or more selected from mannitol, xylitol, dicalcium phosphate, calcium sulfate, starch, lactose, sucrose, dextrose, sorbitol, and cellulose powder. The guanfacine hydrochloride preparation according to an embodiment of the present invention contains 3% by weight to 90% by weight, preferably 10% by weight to 50% by weight of the excipient.

Examples of the disintegrant contained in the guanfacine hydrochloride preparation according to an embodiment of the present invention can include but are not limited to one or more selected from starch, crospovidone, sodium starch glycolate, and croscarmellose sodium. The guanfacine hydrochloride preparation according to an embodiment of the present invention contains 0% by weight to 30% by weight, preferably 0.5% by weight to 20% by weight of the disintegrant.

The sustained-release agent contained in the guanfacine hydrochloride preparation according to an embodiment of the present invention is not particularly limited. The guanfacine hydrochloride preparation according to an embodiment of the present invention contains 15% by weight to 95% by weight, preferably 25% by weight to 70% by weight of the sustained release agent.

Examples of the binder contained in the guanfacine hydrochloride preparation according to an embodiment of the present invention can include but are not limited to one or more selected from polyvinylpyrrolidone, starch, methylcellulose, hydroxypropyl methylcellulose, sucrose solution, dextrose solution, acacia, tragacanth, and locust bean gum. The guanfacine hydrochloride preparation according to an embodiment of the present invention contains 0.2% by weight to 30% by weight, preferably 5% by weight to 15% by weight of the binder.

Examples of the lubricant contained in the guanfacine hydrochloride preparation according to an embodiment of the present invention can include but are not limited to one or more selected from talc, corn starch, silicon dioxide, sodium lauryl sulfate, magnesium stearate, calcium stearate, sodium stearate, stearic acid, sodium stearyl fumarate, hydrogenated cottonseed oil, and waxes (beeswax, carnauba wax, cetyl alcohol, glyceryl stearate, glyceryl palmitate, glyceryl behenate, hydrogenated vegetable oil, and stearyl alcohol). The guanfacine hydrochloride preparation according to an embodiment of the present invention contains 0.2% by weight to 20% by weight, preferably 5% by weight to 15% by weight of the lubricant.

Examples of the colorant contained in the guanfacine hydrochloride preparation according to an embodiment of the present invention can include but are not limited to one or more selected from blue No. 2 aluminum lake and yellow ferric oxide.

### [Evaluation of Stability]

In the present specification, the stability of the guanfacine hydrochloride containing pharmaceutical preparation can be assessed by measuring the amount of related substances using high-performance liquid chromatography (HPLC). 2,6-dichlorophenylacetic acid, which is a hydrolysate, is known as a related substance of guanfacine hydrochloride.

### [Method for Producing Guanfacine Hydrochloride containing Pharmaceutical Preparation]

The method for producing the guanfacine hydrochloride preparation according to the present invention is not particularly limited. In an embodiment, a direct tableting method can be used in which guanfacine hydrochloride, the stability-improving additive, and one or more pharmaceutically acceptable additives are mixed and directly compressed.

### EXAMPLES

### [Comparative Example 1]

Guanfacine hydrochloride 17.1 g, silicified microcrystalline cellulose (PROSOLV HD90, JRS Pharma) 213.9 g, lactose hydrate (Dilactose (registered trademark) S, Freund Corporation) 113 g, crospovidone (Kollidon (registered trademark) CL-F, BASF) 6.00 g, methacrylic acid copolymer (Eudragit (registered trademark) L100-55, Evonik Roehm) 200 g, hydroxypropyl methylcellulose (Methocel (registered trademark) K4M, Colorcon, Inc.) 300 g, glyceryl behenate (Compritol (registered trademark) 888, GATTEFOSSE) 150 g, blue No. 2 aluminum lake 1.00 g, yellow ferric oxide 1.00 g were placed in a plastic bag and mixed. The obtained pharmaceutical composition was tableted using a rotary tableting machine (KIKUSUI SEISAKUSHO LTD.) to obtain a guanfacine hydrochloride preparation of Comparative Example 1.

### [Example 1]

Guanfacine hydrochloride 1.71 g, silicified microcrystalline cellulose (PROSOLV HD90, JRS Pharma) 21.39 g, lactose hydrate (Dilactose (registered trademark) S, Freund Corporation) 6.30 g, crospovidone (Kollidon (registered trademark) CL-F, BASF) 0.60 g, methacrylic acid copolymer (Eudragit (registered trademark) L100-55, Evonik Roehm) 20.0 g, hydroxypropyl methylcellulose (Methocel (registered trademark) K4M, Colorcon, Inc.) 30.0 g, glyceryl behenate (Compritol (registered trademark) 888, GATTEFOSSE) 15.0 g, blue No. 2 aluminum lake 0.10 g, yellow ferric oxide 0.10 g, potassium dihydrogen phosphate (NACALAI TESQUE, INC.) 5.00 g were placed in a plastic bag and mixed. The obtained pharmaceutical composition was tableted using the rotary tableting machine (KIKUSUI SEISAKUSHO LTD.) to obtain a guanfacine hydrochloride preparation of Example 1.

### [Example 2]

A guanfacine hydrochloride preparation of Example 2 was obtained by the same production method as the production method of Example 1 except that lactose hydrate (Dilactose (registered trademark) S, Freund Corporation) was changed to 8.80 g and potassium dihydrogen phosphate (NACALAI TESQUE, INC.) 2.50 g was added.

### [Example 3]

A guanfacine hydrochloride preparation of Example 3 was obtained by the same production method as the production method of Example 1 except that lactose hydrate (Dilactose (registered trademark) S, Freund Corporation) was changed to 3.80 g and potassium dihydrogen phosphate (NACALAI TESQUE, INC.) 7.50 g was added.

### [Example 4]

Guanfacine hydrochloride 1.37 g, silicified microcrystalline cellulose (PROSOLV HD90, JRS Pharma) 17.11 g, lactose hydrate (Dilactose (registered trademark) S, Freund Corporation) 7.04 g, crospovidone (Kollidon (registered trademark) CL-F, BASF) 0.48 g, methacrylic acid copolymer (Eudragit (registered trademark) L100-55, Evonik Roehm) 16.0 g, hydroxypropyl methylcellulose (Methocel (registered trademark) K4M, Colorcon, Inc.) 24.0 g, glyceryl behenate (Compritol (registered trademark) 888, GATTEFOSSE) 12.0 g, blue No. 2 aluminum lake 0.08 g, yellow ferric oxide 0.08 g, phosphoric acid (FUJIFILM Wako Pure Chemical Corporation) 2.00 g were placed in a plastic bag and mixed. The obtained pharmaceutical composition was tableted using the rotary tableting machine (KIKUSUI SEISAKUSHO LTD.) to obtain a guanfacine hydrochloride preparation of Example 4.

### [Example 5]

A guanfacine hydrochloride preparation of Example 5 was obtained by the same production method as the production method of Example 4 except that lactose hydrate (Dilactose (registered trademark) S, Freund Corporation) was changed to 5.04 g and sodium dihydrogen phosphate anhydrous (FUJIFILM Wako Pure Chemical Corporation) 4.00 g was added instead of phosphoric acid.

### [Example 6]

A guanfacine hydrochloride preparation of Example 6 was obtained by the same production method as the production method of Example 5 except that carmellose (NS-300 (registered trademark), NICHIRIN CHEMICAL INDUSTRIES, LTD.) 4.00 g was added instead of sodium dihydrogen phosphate anhydrous.

### [Example 7]

A guanfacine hydrochloride preparation of Example 7 was obtained by the same production method as the production method of Example 5 except that lactose hydrate (Dilactose (registered trademark) S, Freund Corporation) was changed to 5.52 g, carmellose (NS-300 (registered trademark), NICHIRIN CHEMICAL INDUSTRIES, LTD.) 4.00 g was added instead of sodium dihydrogen phosphate anhydrous, and crospovidone was not added.

### [Example 8]

A guanfacine hydrochloride preparation of Example 8 was obtained by the same production method as the production method of Example 4 except that lactose hydrate (Dilactose (registered trademark) S, Freund Corporation) was changed to 7.52 g, carmellose (NS-300 (registered trademark), NICHIRIN CHEMICAL INDUSTRIES, LTD.) 2.00 g was added instead of phosphoric acid, and crospovidone was not added.

### [Example 9]

A guanfacine hydrochloride preparation of Example 9 was obtained by the same production method as the production method of Example 4 except that lactose hydrate (Dilactose (registered trademark) S, Freund Corporation) was changed to 8.72 g, carmellose (NS-300 (registered trademark), NICHIRIN CHEMICAL INDUSTRIES, LTD.) 0.80 g was added instead of phosphoric acid, and crospovidone was not added.

### [Example 10]

A guanfacine hydrochloride preparation of Example 10 was obtained by the same production method as the production method of Example 5 except that microcrystalline cellulose (CEOLUS UF-702, Asahi Kasei Corp.) 4.00 g was added instead of sodium dihydrogen phosphate anhydrous.

### [Example 11]

A guanfacine hydrochloride preparation of Example 11 was obtained by the same production method as the production method of Example 5 except that carmellose calcium (E.C.G-505 (registered trademark), NICHIRIN CHEMICAL INDUSTRIES, LTD.) 4.00 g was added instead of sodium dihydrogen phosphate anhydrous.

### [Example 12]

A guanfacine hydrochloride preparation of Example 12 was obtained by the same production method as the production method of Example 5 except that low-substituted hydroxypropylcellulose (L-HPC (registered trademark) LH-21, Shin-Etsu Chemical Co., Ltd.) 4.00 g was added instead of sodium dihydrogen phosphate anhydrous.

### [Example 13]

A guanfacine hydrochloride preparation of Example 13 was obtained by the same production process as that of Example 5 except that hypromellose phthalate (HPMCP (registered trademark) 55, Shin-Etsu Chemical Co., Ltd.) 4.00 g was added instead of sodium dihydrogen phosphate anhydrous.

### [Comparative Example 2]

A guanfacine hydrochloride preparation of Comparative Example 2 was obtained by the same production method as the production method of Example 5 except that citric acid monohydrate (NACALAI TESQUE, INC.) 4.00 g was added instead of sodium dihydrogen phosphate anhydrous.

### [Comparative Example 3]

A guanfacine hydrochloride preparation of Comparative Example 3 was obtained by the same production method as the production method of Example 5 except that dipotassium hydrogen phosphate (NACALAI TESQUE, INC.) 4.00 g was added instead of sodium dihydrogen phosphate anhydrous.

### [Comparative Example 4]

A guanfacine hydrochloride preparation of Comparative Example 4 was obtained by the same production method as the production method of Example 5 except that croscarmellose sodium (Ac-Di-Sol (registered trademark), DuPont Nutrition Ireland) 4.00 g was added instead of sodium dihydrogen phosphate anhydrous.

### [pH Measurement]

One tablet of the guanfacine hydrochloride preparations of Examples 1 to 13 and Comparative Examples 1 to 4 was pulverized, and 10 ml of water was added. The mixture was mixed with Voltex for 10 seconds, and sonicated for 15 minutes to extract the components contained in the guanfacine hydrochloride preparation. The extract was roughly filtered with a Kimwipe to remove gel-like residues, and then the pH of the filtrate was measured. Measurement results are shown in Table 1.

### [Evaluation of Stability]

The guanfacine hydrochloride preparations of Examples 1 to 13 and Comparative Examples 1 to 4 were stored at 60°C and 60% relative humidity open conditions for 1 week or 2 weeks. The initial value before storing (initial) and the amount (%) of the related substance (hydrolysate) at the time of storing were measured. One tablet before storing (immediately after production) and after storing was pulverized using an agate mortar, and about 8 ml of acetonitrile : water mixture (4 : 1) was added and stirred. In addition, acetonitrile / water mixture (4 : 1) was added to make exactly 10 ml. This solution was filtered through a membrane filter with a pore size of 0.45 µm or less, and 3 ml or more of the first filtrate was removed, and the next filtrate was used as a sample solution.

The amount of related substances was measured by an HPLC method, taking exactly 2 µl of the above sample solution. By the area percentage method, the sum of the peak areas of guanfacine and related substances derived from guanfacine obtained on a chromatogram was taken as 100, and the amount (%) of 2,6-dichlorophenylacetic acid, which is a main related substance derived from guanfacine hydrochloride, was calculated from the ratio of the peak areas.

The values of hydrolysate of guanfacine hydrochloride are shown in Table 1.

**[Table 1]**

| | Comparative Example 1 | Example 1 | Example 2 | Example 3 |
|---|---|---|---|---|
| pH | 5.78 | 4.25 | 4.55 | 4.66 |
| Initial | 0.06 | 0.06 | N.D. | 0.02 |
| 60°C, 60%RH, open 1W | 0.42 | 0.15 | 0.13 | 0.15 |
| 60°C, 60%RH, open 2W | 0.75 | 0.31 | 0.27 | 0.28 |

| | Example 4 | Example 5 | Example 6 | Example 7 |
|---|---|---|---|---|
| pH | 2.98 | 4.53 | 4.18 | 4.53 |
| Initial | N.D. | N.D. | N.D. | N.D. |
| 60°C, 60%RH, open 1W | 0.09 | 0.13 | 0.04 | 0.03 |
| 60°C, 60%RH, open 2W | 0.22 | 0.31 | 0.09 | 0.07 |

| | Example 8 | Example 9 | Example 10 | Example 11 |
|---|---|---|---|---|
| pH | 4.44 | 4.57 | 4.72 | 5.61 |
| Initial | N.D. | N.D. | N.D. | N.D. |
| 60°C, 60%RH, open 1W | 0.04 | 0.08 | 0.12 | 0.17 |
| 60°C, 60%RH, open 2W | 0.07 | 0.11 | 0.27 | 0.33 |

| | Example 12 | Example 13 | Comparative Example 2 | Comparative Example 3 |
|---|---|---|---|---|
| pH | 5.68 | 4.37 | 2.98 | 6.90 |
| Initial | N.D. | N.D. | N.D. | N.D. |
| 60°C, 60%RH, open 1W | 0.17 | 0.13 | 0.28 | 2.13 |
| 60°C, 60%RH, open 2W | 0.26 | 0.20 | 0.67 | 5.28 |

| | Comparative Example 4 | | | |
|---|---|---|---|---|
| pH | 6.12 | | | |
| Initial | N.D. | | | |
| 60°C, 60%RH, open 1W | 0.54 | | | |
| 60°C, 60%RH, open 2W | 1.05 | | | |

From the results shown in Table 1, in the guanfacine hydrochloride preparations of Comparative Example 3 in which pH was 6.90, and Comparative Example 4 in which pH was 6.12, a significant increase in the hydrolysate of guanfacine hydrochloride was observed. On the other hand, due to the effect of the stability-improving additive, the amount of hydrolysate of guanfacine hydrochloride was reduced in the guanfacine hydrochloride preparations of Examples 1 to 5 and 10 to 13 with a lower pH than in Comparative Example 1 in which the additive is not contained. However, in the guanfacine hydrochloride preparation of Comparative Example 2 in which the pH was 2.98, an increase in the hydrolysate of guanfacine hydrochloride was observed. In addition, in the guanfacine hydrochloride preparations of Examples 6 to 9 containing carmellose as the stability-improving additive, an increase in the hydrolysate of guanfacine hydrochloride was significantly suppressed.

## Claims

1. A guanfacine hydrochloride preparation comprising:
guanfacine hydrochloride; and
one or more stability-improving additives selected from a group consisting of potassium dihydrogen phosphate, phosphoric acid, sodium dihydrogen phosphate anhydrous, carmellose, microcrystalline cellulose, carmellose calcium, low-substituted hydroxypropylcellulose, and hypromellose phthalate.

2. The guanfacine hydrochloride preparation according to claim 1, wherein
0.5% by weight to 25% by weight of the stability-improving additive is contained with respect to 100% by weight of the guanfacine hydrochloride preparation.
